# EUROPÄISCHE PATENTSCHRIFT

(11) **EP 1 282 715 B1**
(45) Veröffentlichungstag und Bekanntmachung des Hinweises auf die Patenterteilung: **21.07.2004**
(21) Anmeldenummer: 01943410.9
(22) Anmeldetag: 18.05.2001
(51) Int. Cl.: C12N 15/54, C12N 9/10, C12N 15/63, C12N 1/21, C12N 1/20, C12P 19/12

(54) **VERFAHREN ZUR MIKROBIELLEN HERSTELLUNG VON DIFRUCTOSEANHYDRID-III, DAFÜR EINSETZBARER MIKROORGANISMUS SOWIE ENZYM MIT INULASE-II-AKTIVITÄT UND DAFÜR KODIERENDE DNA-SEQUENZEN**
METHOD FOR MICROBIAL PRODUCTION OF DIFRUCTOSE ANHYDRIDE III, MICRO-ORGANISM USED THEREFOR AND ENZYME WITH INULASE II ACTIVITY AND DNA SEQUENCES CODING THEREFOR
PROCEDE DE FABRICATION MICROBIENNE D'ANHYDRIDE DE DIFRUCTOSE III, MICRO-ORGANISME DESTINE A CET EFFET, ENZYME AYANT UNE ACTIVITE D'INULASE II ET SEQUENCES D'ADN CODANT CETTE ENZYME

(30) Priorität: 19.05.2000 DE 10024569
(43) Veröffentlichungstag der Anmeldung: 12.02.2003
(73) Patentinhaber: Nordzucker AG, 38100 Braunschweig (DE)
(72) Erfinder: WALTER, Martin, 38176 Bortfeld (DE); SCHUBERT, Milada, 38102 Braunschweig (DE); VORLOP, Klaus-Dieter, 38102 Braunschweig (DE); JAHNZ, Ulrich, 38106 Braunschweig (DE)
(74) Vertreter: Einsel, Martin
(86) Internationale Anmeldenummer: PCT/EP2001/005737
(87) Internationale Veröffentlichungsnummer: WO 2001/090370

(56) Entgegenhaltungen:
- EP-A- 0 332 108
- DE-A- 19 547 059
- SAKURAI HIROAKI ET AL: "Molecular cloning of an Inulin fructotransferase (Depolymerizing) gene from Arthrobacter sp. H65-7 and its expression in Escherichia coli." BIOSCIENCE BIOTECHNOLOGY AND BIOCHEMISTRY, Bd. 61, Nr. 1, 1997, Seiten 87-92, XP002192974 ISSN: 0916-8451
- YOKOTA A ET AL: "PURIFICATION AND PROPERTIES OF AN INULIN FRUCTOTRANSFERASE DEPOLYMERIZING FROM ARTHROBACTER-SP H65-7" JOURNAL OF FERMENTATION AND BIOENGINEERING, Bd. 72, Nr. 4, 1991, Seiten 262-265, XP001064402 ISSN: 0922-338X

## Beschreibung

Die vorliegende Erfindung betrifft ein Verfahren zur mikrobiellen Herstellung von Difructoseanhydrid-III, einen für dieses Verfahren geeigneten Mikroorganismus mit der Fähigkeit, ein Enzym mit Inulase-II-Aktivität zu exprimieren, ein Enzym mit Inulase-II-Aktivität sowie DNA-Sequenzen mit einem für dieses Enzym kodierenden Bereich.

Difructroseanhydrid-III ist ein Disaccharid, das zwei Fructoseeinheiten enthält, die über 1-2' und 2-3'-Bindungen miteinander verknüpft sind.
Difructoseanhydrid-III (DFA-III) kann durch mikrobiellen Abbau von Inulin durch das Enzym Inulase II, einer Transferase, gewonnen werden.

Es ist bekannt, dass das Enzym Inulase II von einigen Mikroorganismen produziert werden kann. Dazu gehören verschiedene Spezies der Gattung Arthrobacter, wie zum Beispiel Arthrobacter ureafaciens 7116, Arthrobacter globiformis C 11-1, Arthrobacter aurescens IFO 12136 und Arthrobacter ilicis MCI-2297 sowie der Gattung Pseudomonas wie Pseudomonas fluorescens Nr. 949.

Ein Verfahren zum mikrobiellen Abbau von Inulin zu DFA-III mittels Arthrobacter ilicis ist in EP 0 332 108 B1 beschrieben, wobei das aus diesem Mikroorganismus erhaltene Enzym mit Inulase-II-Aktivität maximale Aktivität bei 60 °C zeigt und für kurze Zeit bis zu einer Temperatur von 70 °C stabil ist. Angaben über die Zeitdauer sowie die Restaktivität finden sich jedoch nicht.

Dokument D1 (Sakurai Hiroaki et al. (1997) Bioscience Biotechnology and Biochemistry, Bd. 61, Nr.1, Seiten 87-92) Offenbart die Nukleinsäuresequenz von ift,welche für das Enzym Inulase II von Arthrobacter sp. H65-7 kodiert. Die ift Sequenz wurde in den Vektor pUC19 kloniert und in E. coli exprimiert. Das Enzym wurde für die Herstellung von Difructoseanhydrid-III (DFAIII) ausgehend von Inulin verwendet. Diese Inulase II aus Arthrobacter sp. H65-7 wurde bereits vorher isoliert und characterisiert (D2 : Yokota A. et al. (1991)

Journal of Fermentation and Bioengineering, Bd.72, (Nr.4, Seiten 262-265). D2 wird auch als Referenz in D1 angegeben. Dokument D2 offenbart eine maximale Aktivität dieses Enzyms bei 60°C. Es wurde des weiteren geziegt ,daß das Enzym bis 70°C stabil ist.

Es bestand jedoch Bedarf nach weiteren verbesserten Verfahren, insbesondere nach Verfahren, die mit einfach erhältlichen und gängigen (rekombinanten) Mikroorganismen durchgeführt werden können sowie nach Enzymen, die selbst bei erhöhter Temperatur über einen langen Zeitraum zum Beispiel mehrere Stunden noch hohe Restaktivitäten, vorzugsweise von bis zu 100 %, aufweisen.

Es war daher Aufgabe der vorliegenden Erfindung, ein Verfahren zur mikrobiellen Herstellung von Difructoseanhydrid-III bereitzustellen, dass mit einfach erhältlichen gängigen Mikroorganismen durchführbar ist, die mit hoher Effizienz DFA-III aus Inulin gewinnen können.

Weiter war es Aufgabe der Erfindung, ein Enzym mit Inulase-II-Aktivität bereitzustellen, das über einen langen Zeitraum eine hohe Thermostabilität aufweist.

Die vorliegende Erfindung stellt zur Lösung der Aufgabe insbesondere DNA-Sequenzen, die für ein Enzym mit inulase-II-Aktivität kodieren, sowie Mikroorganismen, die dieses Gen enthalten und exprimieren können, zur Verfügung, die für ein Verfahren zur mikrobiellen Herstellung von DFA-III vorteilhaft verwendet werden können.

Gegenstand der vorliegenden Erfindung sind daher DNA-Sequenzen, die für ein Enzym mit Inulase-II-Aktivität kodieren, dadurch gekennzeichnet, dass es nach Einführung dieser DNA-Sequenzen in einen Mikroorganismus zur Expression des Enzyms mit Inulase-II-Aktivität kommt, das den Abbau von Inulin zu DFA-III bewirkt.

Gegenstand der Erfindung sind insbesondere DNA-Sequenzen, die für ein Enzym mit Inulase-II-Aktivität kodieren, umfassend
eine Nucleotidabfolge gemäß Sequenz Nr. 1, Sequenz Nr. 2 oder Sequenz Nr. 3 wie sie in den Figuren dargestellt sind;
eine Nucleotidabfolge, die den für ein Enzym mit Inulase-II-Aktivität kodierenden Bereich gemäß einer der Sequenzen Nrn. 1 bis 3 umfasst, sowie
eine Nucleotidabfolge, die für ein Enzym kodiert, das die für die Sequenzen Nrn. 1 bis 3 angegebenen Aminosäuresequenzen umfasst.

Eine Wiedergabe der Sequenzen findet sich in dem Sequenzprotokollteil der Beschreibung:
- DNA-Sequenz Nr. 1 mit der davon abgeleiteten Aminosäuresequenz,
- DNA-Sequenz Nr. 2 mit der davon abgeleiteten Aminosäuresequenz, und
- DNA-Sequenz Nr. 3.

Gegenstand der Erfindung ist ferner ein Mikroorganismus der Gattung Arthrobacter, der eine der vorstehend genannten DNA-Sequenzen enthält, sowie Plasmide und rekombinante Mikroorganismen, die eine der vorstehend genannten DNA-Sequenzen enthalten.

Darüber hinaus ist Gegenstand der Erfindung ein Verfahren zur mikrobiellen beziehungsweise enyzmatischen Herstellung von Difructoseanhydrid-III, das unter Verwendung einer der vorstehend genannten DNA-Sequenzen beziehungsweise eines Plasmids oder eines Mikroorganismusses, die eine der vorstehend genannten DNA-Sequenzen enthalten, durchgeführt wird.

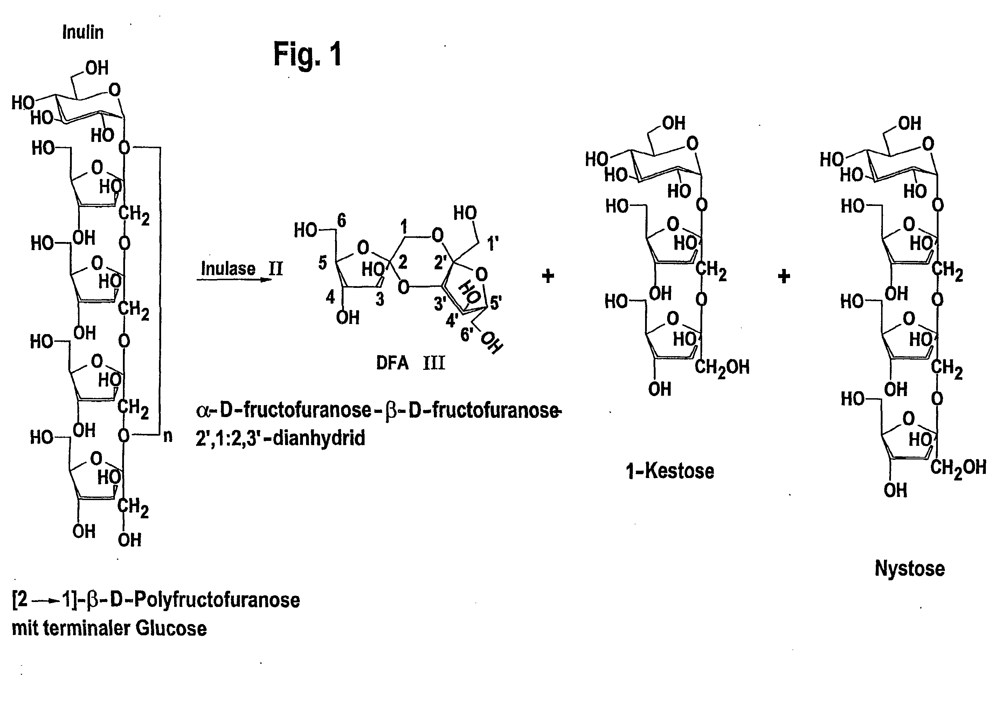

Es zeigen
- **Figur 1**: die enzymatische Synthese von DFA-III und Fructooligosacchariden aus Inulin;
- **Figur 2**: die Genkarte der Bam H1-Fragmente MSiftBH2 und MSiftBH1 aus Arthrobacter Bu0141;
- **Figur 3**: die DNA-Sequenz Nr. 1 und die davon abgeleitete Aminosäuresequenz der Expressionsmatrize MSiftPH mit dem für aktive Inulase-II kodierenden Bereich;
- **Figur 4**: die Genkarte des Plasmids pMSiftPH sowie davon abgeleitete modifizierte DNA-Sequenzen, die für Inulase-II kodieren;
- **Figur 5**: die Genkarte des Plasmids pMSiftOptWT;
- **Figur 6**: die DNA-Sequenz Nr. 2 der Expressionsmatrize MSiftOptWT und die davon abgeleiteten Aminosäuresequenz; und
- **Figur 7**: die DNA-Sequenz Nr. 3 des Plasmids pMSiftOptR.

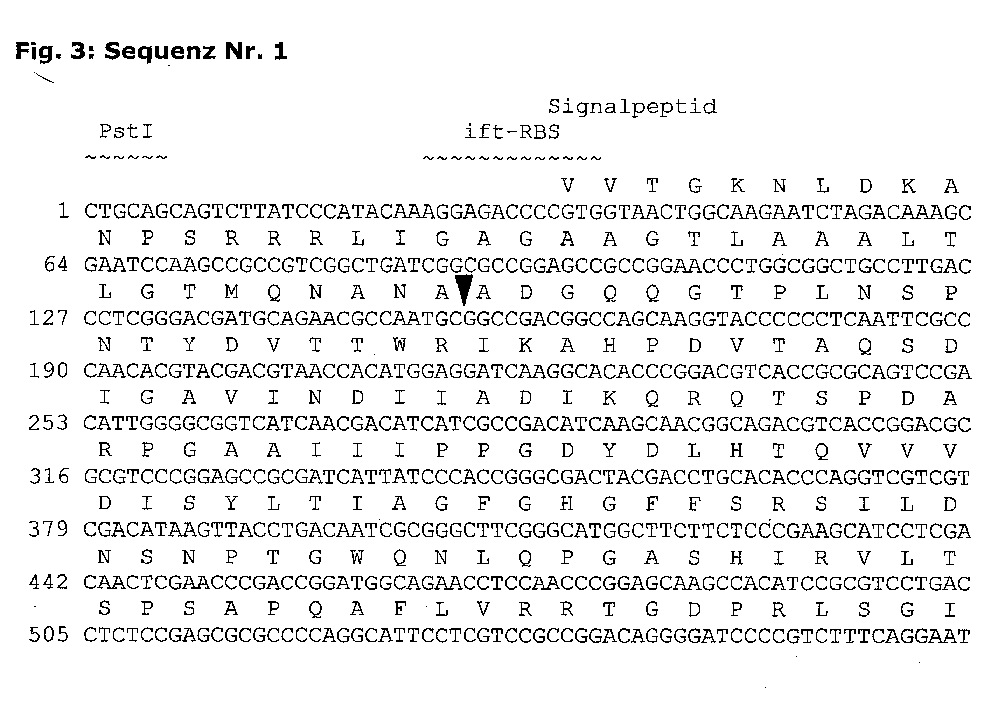

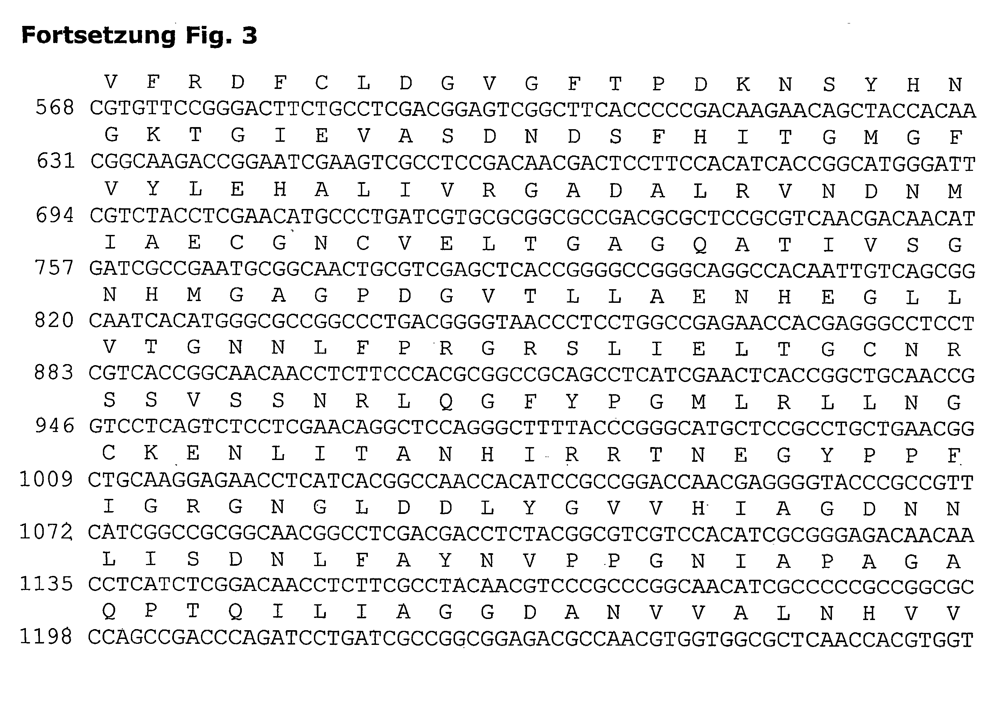

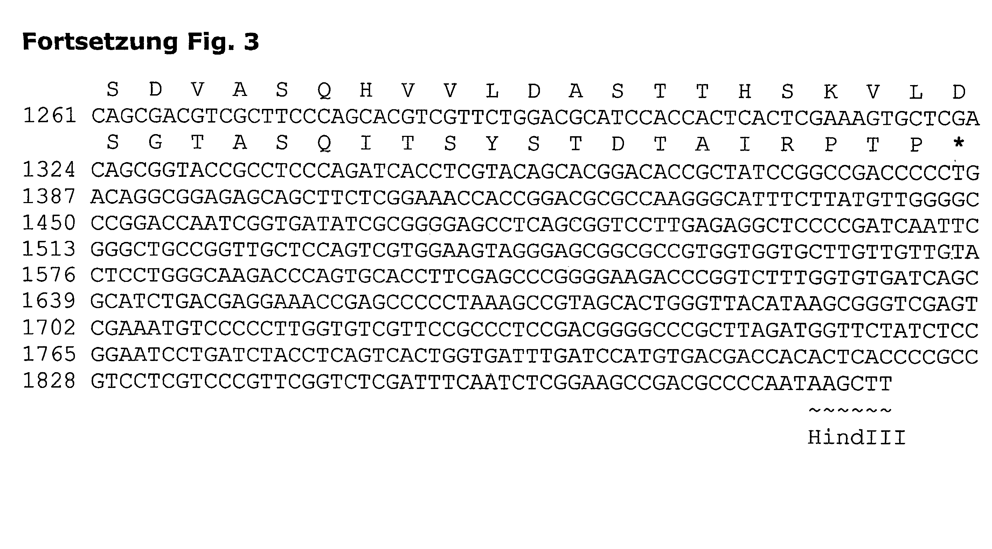

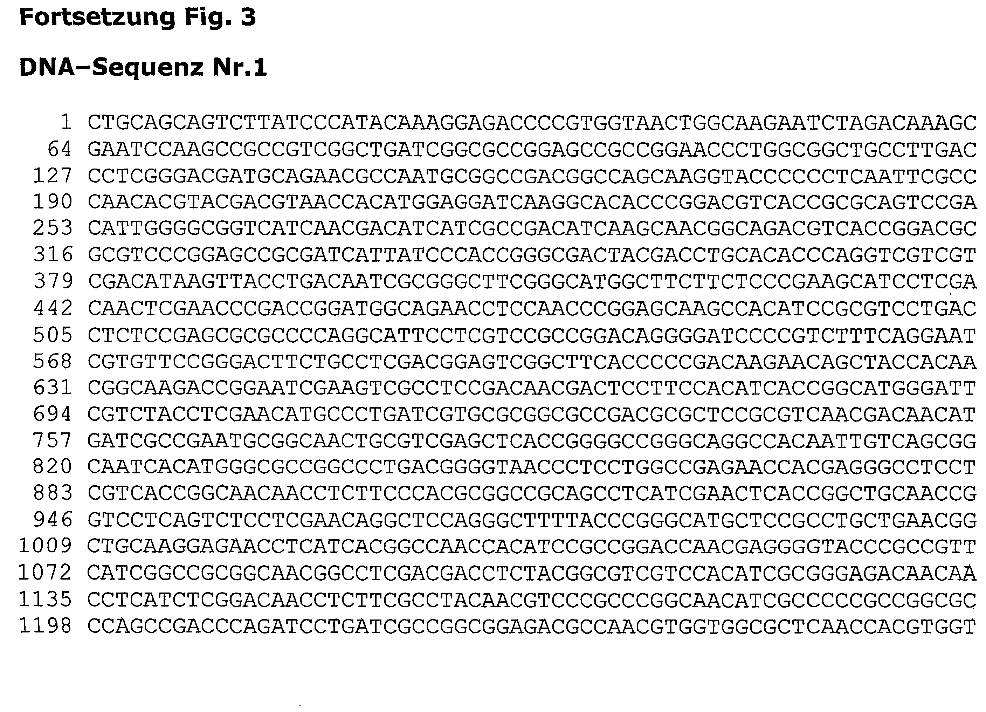

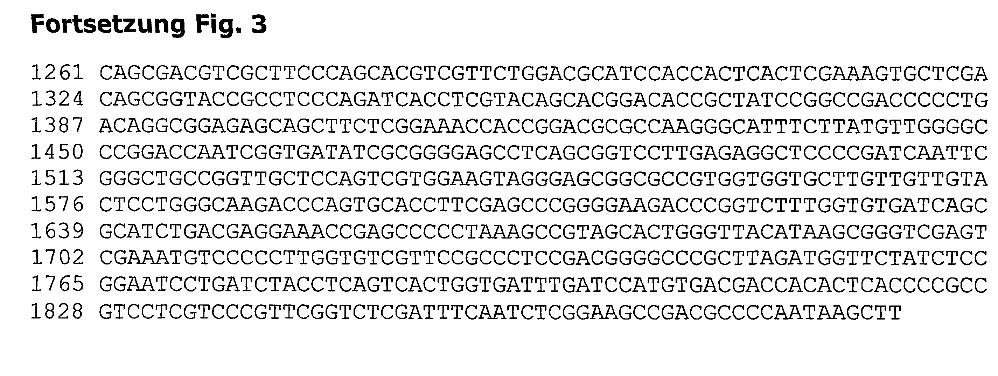

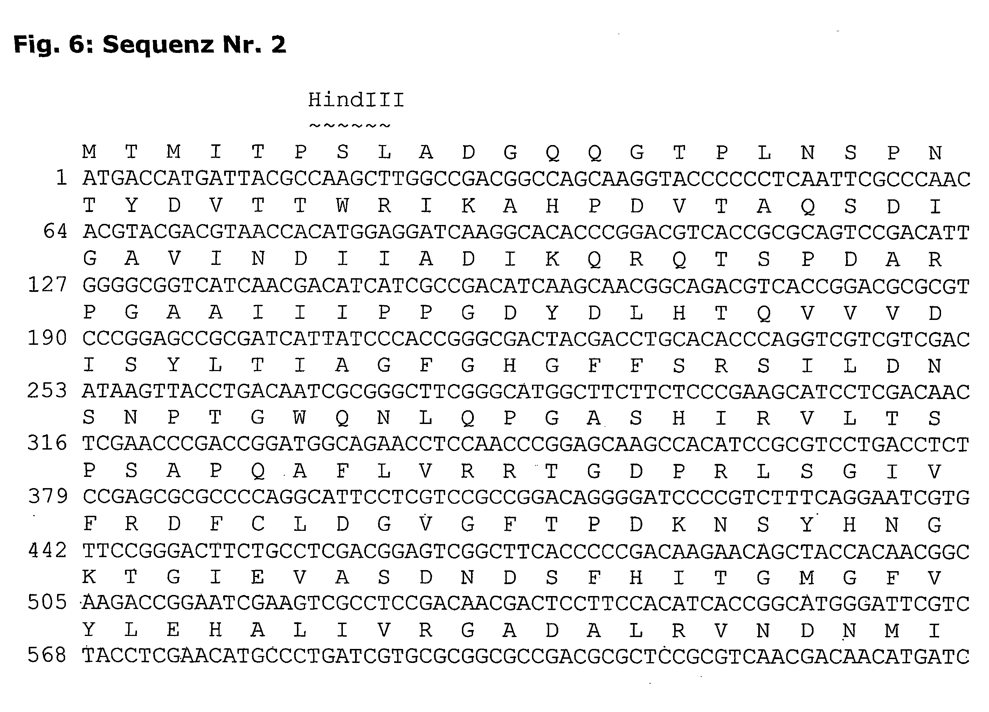

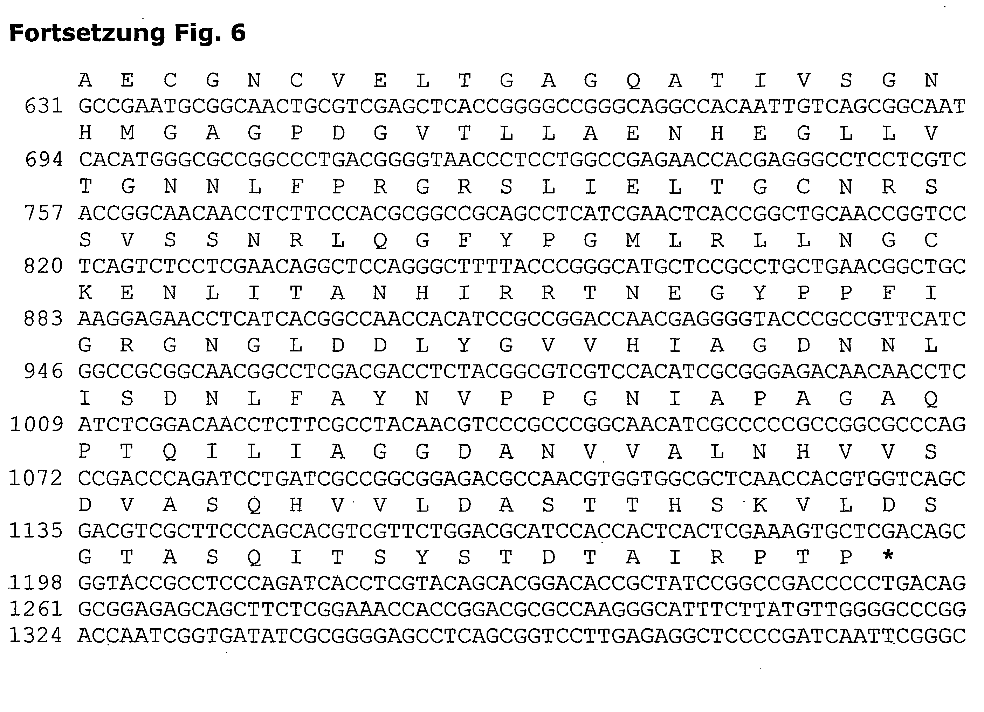

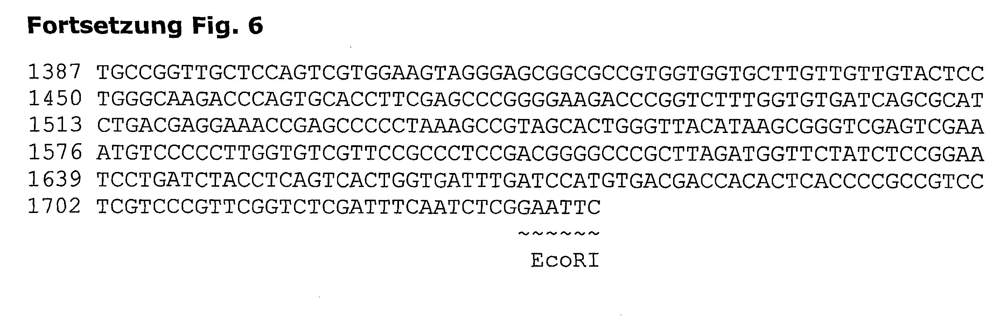

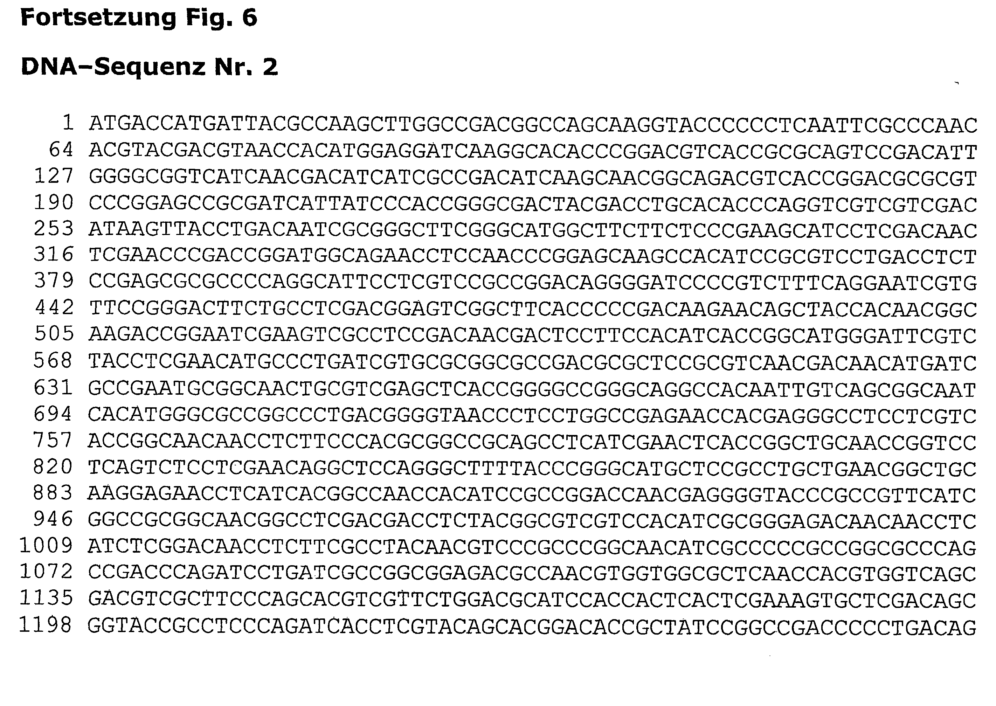

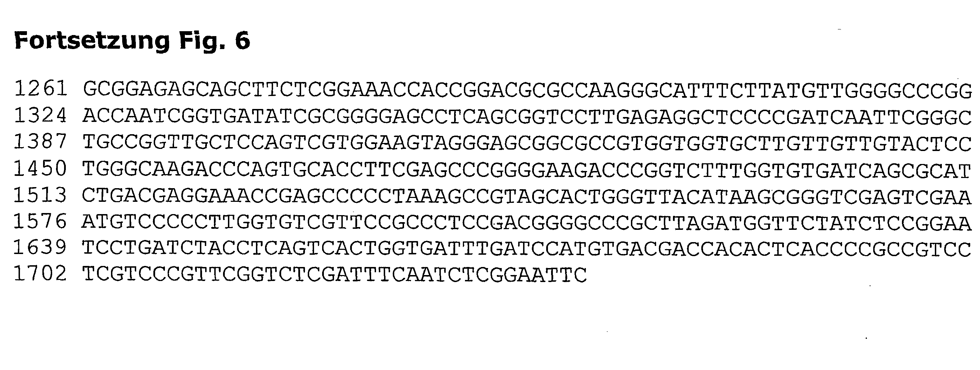

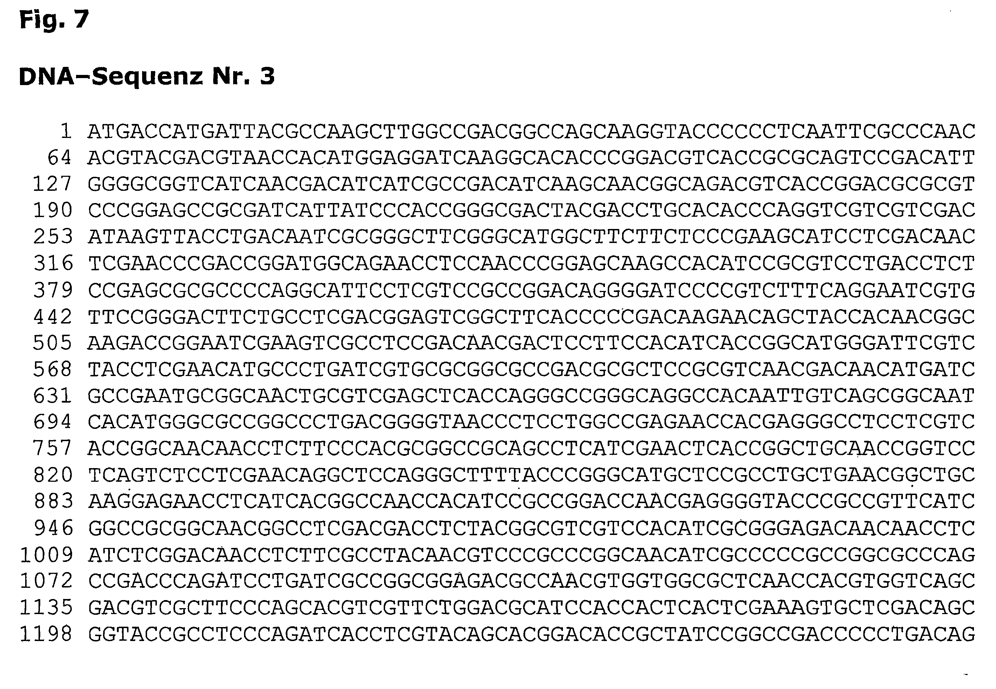

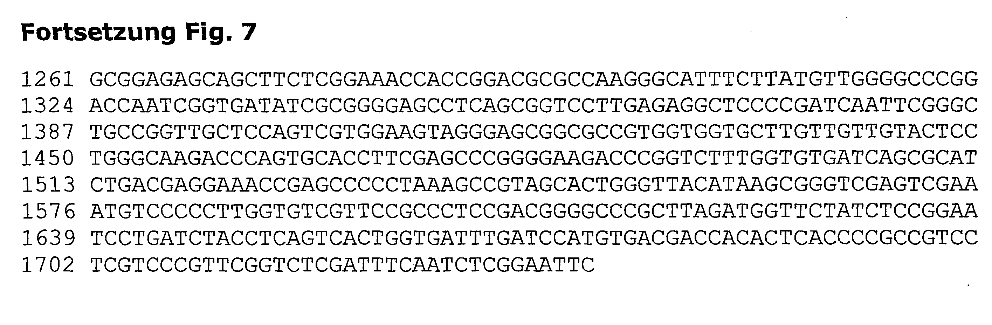

In den Fortsetzungen der Figuren 3 und 6 sowie in Figur 7 ist zudem der kodierende Strang in 5'-3'-Richtung (von links nach rechts) in einer separaten Darstellung gezeigt.

Von der vorliegenden Erfindung werden auch DNA-Sequenzen umfasst, die zum Beispiel ein Fragment, Derivat oder allelische Variante der vorstehend beschriebenen DNA-Sequenzen darstellen, die für ein Enzym mit Inulase-II-Aktivität kodieren. Der Ausdruck Derivat bedeutet in diesem Zusammenhang, dass die Sequenzen sich von den vorstehend beschriebenen DNA-Sequenen an einer oder mehreren Positionen unterscheiden, aber einen hohen Grad an Identität zu diesen Sequenzen aufweisen. Ein hoher Grad an Identität bedeutet dabei eine Sequenzidentität über 90 % und insbesondere von mindestens 95 % für die Sequenz einschließlich der Signalsequenz und/oder für die Sequenz der reifen Untereinheit, wobei besagte Sequenzen für ein Enzym mit Inulase-II-Aktivität kodieren, wobei das Enzym bei 60°C über 140 Stunden bei 100% Restaktivität stabil ist.
Weiter umfasst die vorliegende Erfindung auch DNA-Sequenzen, deren komplementärer Strang mit einer der vorstehend genannten erfindungsgemäßen DNA-Sequenzen hybridisiert und die für ein Enzym mit Inulase-II-Aktivität kodieren.
Im Rahmen der vorliegenden Erfindung bedeutet der Begriff "Hybridisierung" eine Hybridisierung unter konventionellen Hybridisierungsbedingungen. Vorzugsweise wird darunter eine Hybridisierung unter stringenten Bedingungen verstanden.

insbesondere fallen unter die Erfindung DNA-Sequenzen, die den für die reife Untereinheit kodierenden Bereich gemäß einer der Sequenzen Nrn. 1 bis 3 beziehungsweise eine wie vorstehend beschriebene Modifikation davon aufweisen.

Die in Sequenz Nr. 1 dargestellte DNA-Sequenz ist eine genomische Sequenz, die einen kodierenden Bereich für ein Enzym mit Inulase-II-Aktivität aus einem Mikroorganismus Arthrobacter sp. Bu0141 umfasst.
Mit Hilfe dieser Sequenzen ist es dem Fachmann nun möglich, homologe Sequenzen aus anderen Arthrobacterarten oder -stämmen zu isolieren. Dies kann beispielsweise mit Hilfe konventioneller Methoden, wie dem Durchmustern von Genbanken mit geeigneten Hybridisierungssonden erfolgen.

Die erfindungsgemäßen DNA-Sequenzen kodieren ein Enzym mit Inufase-II-Aktivität.

Der Mikroorganismus Arthrobacter sp. Bu0141, die vorstehend genannten Plasmide sowie rekombinante E.coli mit Plasmiden pMSiftOptWT und pMSiftOptR wurden bei der Deutschen Sammlung von Mikroorganismen und Zellkulturen GmbH unter den folgenden Nummern hinterlegt und sind ebenfalls Gegenstand der Erfindung:

| | | |
|---|---|---|
| Plasmid | pMSiftPH | DSM 13460 |
| Plasmid | pMSiftOptR | DSM 13461 |
| Plasmid | pMSiftOptWT | DSM 13462 |
| E.coli | pMSiftOptWT | DSM 13463 |
| Arthrobacter | sp.Bu0141 | DSM 13464 |
| E.coli | pMSiftOptR | DSM 13465 |

Dieser Arthrobacter-Stamm, nachfolgend als Bu0141 bezeichnet, wurde aus einer Bodenprobe isoliert und konnte keiner der bisher beschriebenen Arten zugeordnet werden. Die Eigenschaften des Stammes Bu0141 werden nachfolgend näher beschrieben.

Der Mikroorganismus bildet coryneforme Stäbchen, ist gram-positiv, strikt aerob und bildet keine Säure oder Gas aus Glucose.

| | |
|---|---|
| Beweglichkeit | + |
| Sporen | - |
| Katalase | + |
| Meso-Diaminopimelinsäure in der Zellwand: | nein |
| Peptidoglycan-Typ | A3α, L-Lys-L-Ala_{2.3} |

Die Sequenzierung des Bereiches mit der größten Variabilität (16S rDNA-Sequenz) ergab als höchsten Wert 97,8 % Übereinstimmung mit Arthrobacter globiformis. Die mehr als 2 % Unterschiede in den 16S rDNA-Sequenzen lassen darauf schließen, dass es sich bei dem Mikroorganismus um einen Vertreter einer zwar mit A.globiformis nahe verwandten, bisher aber noch nicht beschriebenen Art handelt, die zudem nicht pathogen ist.

Es hat sich gezeigt, dass dieser Stamm ein Enzym mit Inulase-II-Aktivität produzieren kann, das über lange Zeit bei erhöhter Temperatur stabil ist. So wurde festgestellt, dass das Enzym bei 60 °C über 140 Stunden bei 100 % Restaktivität stabil ist.

Aus diesem Arthrobacter sp. Bu0141 wurde eine DNA-Sequenz (Sequenz Nr. 1) isoliert, die den für das Enzym mit Inulase-11-Aktivität kodierenden Bereich umfasst.

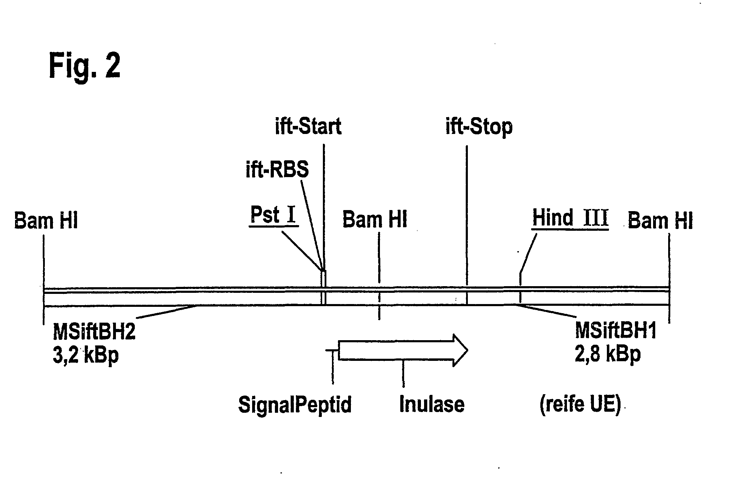

Zur Isolierung wurde das ift-Gen (kodiert für Inulase) aus dem Arthrobacter sp. Bu0141 in λ-Phagen kloniert, in E.coli subkloniert und in seiner kompletten Länge auf zwei Bam H1-Fragmenten isoliert. Die Genkarte dieser Fragmente, die MSiftBH2 und MSiftBH1 bezeichnet wurden, ist in Figur 2 gezeigt.

Das Fragment MSiftBH2 hat eine Länge von zirka 3,2 kBp, wobei ein Teil für die N-terminale Hälfte des ift-Gens kodiert. Das Fragment MSiftBH1 hat eine Länge von ca. 2,8 kBp, wobei ein Teil für die C-terminale Hälfte des ift-Gens kodiert. Die beiden Bam H1-Fragmente wurden aus der gesamten genomischen DNA von Arthrobacter sp. Bu0141 isoliert.

In der in Figur 2 dargestellten Genkarte sind die singulären Restriktionsstellen Pst I und Hind III angegeben, die wie nachstehend beschrieben zur Konstruktion einer Expressionsmatrize dienen. Weiter sind in der Genkarte die mutmaßliche Ribosomenbindungsstelle ift-RBS sowie das Start- und Stopp-Codon markiert (ift-Start beziehungsweise ift-Stopp), die den kodierenden Bereich begrenzen.

Figur 3 zeigt die DNA-Sequenz Nr. 1 und darüber die davon abgeleitete Aminosäuresequenz des in der Genkarte in Figur 2 gekennzeichneten Pst I/Hind III-Fragments mit dem ift-Gen und seiner Umgebung aus Arthrobacter sp. Bu0141. Dieses Fragment wird nachstehend als Expressionsmatrize MSiftPH bezeichnet.

Die Expressionsmatrize MSiftPH enthält 1884 Nucleotide.
Das Enzym mit Inulase-II-Aktivität wird von 1350 Nucleotiden kodiert und besteht aus 450 Aminosäuren. Die ersten 40 Aminosäuren dienen als Signalpeptid und gewährleisten den Transport des exprimierten Enzyms aus der Zelle. Dieses Signalpeptid wird während oder nach dem Transport des Enzyms aus der Zelle in Arthrobacter sp. Bu0141 abgeschnitten. Die reife Untereinheit des Enzyms mit Inulase-II-Aktivität selbst besteht aus 410 Aminosäuren.

In der in Figur 3 gezeigten Sequenz sind zudem die mutmaßliche Ribosomenbindungsstelle ift-RBS mit dem Start-Codon GTG, das Stopp-Codon (*) und die mutmaßliche Schnittstelle zwischen dem Signalpeptid und dem kodierenden Bereich der reifen Untereinheit (▼) gekennzeichnet.

Ausgehend von dem Fragment MSiftPH wurden nun erfindungsgemäß Fremdexpressionssysteme entwickelt, die in einen Wirtsorganismus eingeführt werden und die Expression eines Enzyms mit Inulase-II-Aktivität in diesem Wirtsorganismus bewirken können.

Hierzu wurde das vorstehende DNA-Fragment MSiftPH beziehungsweise Teile davon, die den kodierenden Bereich für das Enzym mit Inulase-II-Aktivität enthalten, mit den für den jeweiligen Wirtsorganismus geeigneten die Transkription steuernden Elementen, wie Promotor und Stopp-Codon verknüpft, wobei die DNA-Sequenz bei Bedarf vor oder nach der Verknüpfung modifiziert werden kann.

Beispielsweise wurde die Signalsequenz ganz beziehungsweise teilweise aus der kodierenden DNA entfernt, da diese in der Regel von einem Wirtsorganismus weder zum Export aus der Zelle erkannt noch posttranslational abgespalten werden kann.

Hierbei hat sich gezeigt, dass durch Verkürzung beziehungsweise komplette Entfernung der Signalsequenz eine deutliche Steigerung der Enzymaktivität bewirkt werden kann. Die Ergebnisse dieser Deletionsversuche sind nachstehend beschrieben.
Mit Hilfe der unter Figur 3 dargestellten DNA-Sequenz 1 beziehungsweise Teilen davon, die den kodierenden Bereich für das Enzym mit Inulase-II-Aktivität enthalten, ist es möglich, Mikoorganismen dahingehend zu verändern, dass sie aktive Inulase-II exprimieren.

Zur Vorbereitung der Einführung fremder Gene in Mikroorganismen stehen eine große Anzahl von Klonierungsvektoren zur Verfügung, die die für einen bestimmten Mikroorganismus benötigten Elemente zur Steuerung der Expression enthalten. Die gewünschte Sequenz kann an einer passenden Restriktionsschnittstelle in den Vektor eingeführt werden. Hierbei kann jede Plasmid-DNA-Sequenz in den gleichen Vektor oder in andere Plasmiden kloniert werden. Die Techniken, Vektoren und entsprechenden Steuerungselemente sind an sich bekannt und können ohne weiteres für den jeweils zu transformierenden Wirtsorganismus ausgewählt und/oder angepasst werden.

Nachstehend wird die Erzeugung von erfindungsgemäßen rekombinanten Wirtsorganismen, die eine erfindungsgemäße DNA-Sequenz enthalten und die Fähigkeit haben, aktive Inulase-II zu exprimieren, am Beispiel der Transformation von E.coli beschrieben, wobei in den Mikroorganismus Expressionskonstrukte eingeführt wurden, die die in Figur 3 gezeigte DNA-Sequenz 1 beziehungsweise Teile davon mit dem für Inulase-II kodierenden Bereich enthalten. Als Vektoren wurden für das nachstehende Beispiel pUC 18 sowie pUC 19 verwendet. In diese Vektoren wurde das DNA-Fragment MSiftPH gemäß Figur 3 und Modifikationen davon, die den für aktive Inulase-II kodierenden Bereich enthielten, eingeführt und die entsprechende Enzymaktivität damit transformierter E.coli untersucht.

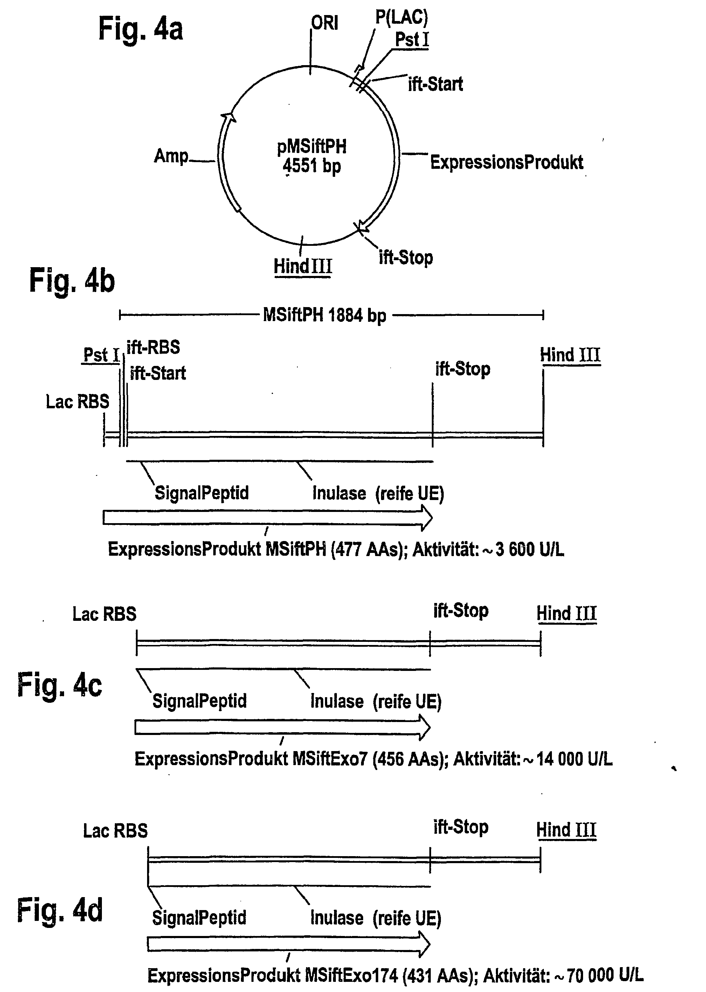

Figur 4 zeigt die Genkarte des erhaltenen Inulase-Expressionskonstruktes pMSiftPH, wobei die Expressionsmatrize MSiftPH, die in Figur 3 gezeigt ist, in den käuflichen Vektor pUC 18 integriert worden ist. Das Expressionskonstrukt pMSiftPH wurde in E.coli transformiert. Die Qualität des Expressionskonstrukts wurde in dem nachstehend beschriebenen Inulase-Aktivitätstest kontrolliert. Transformanten mit dem Expressionskonstrukt pMSiftPH zeigten eine deutliche Inulaseaktivität von etwa 3600 U/L.

Es wurden Deletionsversuche unternommen, um den Einfluss des Signalpeptids auf die Enzymaktivität zu untersuchen. Es hat sich hierbei gezeigt, dass durch Verkürzung des Signalpeptids auf dem DNA-Niveau die Inulaseaktivität des Expressionskonstrukts pMSiftPH auf das 20-fache gesteigert werden konnte. So zeigt ein Expressionsprodukt (Enzym) mit lediglich 456 Aminosäuren gegenüber 477 Aminosäuren für das Expressionsprodukt (Enzym) von MSiftPH eine Aktivität von etwa 14.000 U/L (Figur 4c) und das entsprechende Expressionsprodukt (Enzym) mit 431 Aminosäuren eine Aktivität von etwa 70.000 U/L (Figur 4d).

Eine DNA-Sequenz (Sequenz Nr. 2), die für aktive Inulase-II kodiert, bei der die für das Signalpeptid kodierende DNA-Sequenz vollständig entfernt worden ist, ist in Figur 6 gezeigt. Die in Figur 6 gezeigte Nucleotidsequenz Nr. 2 wird nachstehend als Expressionsmatrize MSiftOptWT bezeichnet. In dieser Sequenz beginnt der für die reife Inulase-Untereinheit ohne Signalpeptid kodierende Bereich an der Nucleotidposition 25.

Die Expressionsmatrize MSiftOptWT wurde in mehreren Vektoren auf Effizienz getestet. Hierbei hat sich gezeigt, dass allein durch Umklonieren derselben Expressionsmatrize von dem Plasmid pUC 18 nach pUC 19 eine Steigerung der Inulase-Aktivität um den Faktor 2 bis 3 erzielt werden konnte.

Das Expressionskonstrukt pMSiftOptWT wurde aus der Expressionsmatrize MSiftOptWT und dem Plasmid pUC 19 hergestellt, indem das MSiftOptWT-Fragment nucleotidgenau optimiert über die künstlich erzeugten Hind III- beziehungsweise Eco RI-Schnittstellen direkt in das Leseraster, das an der Lac RBS beginnt, in pUC 19 integriert wurde.

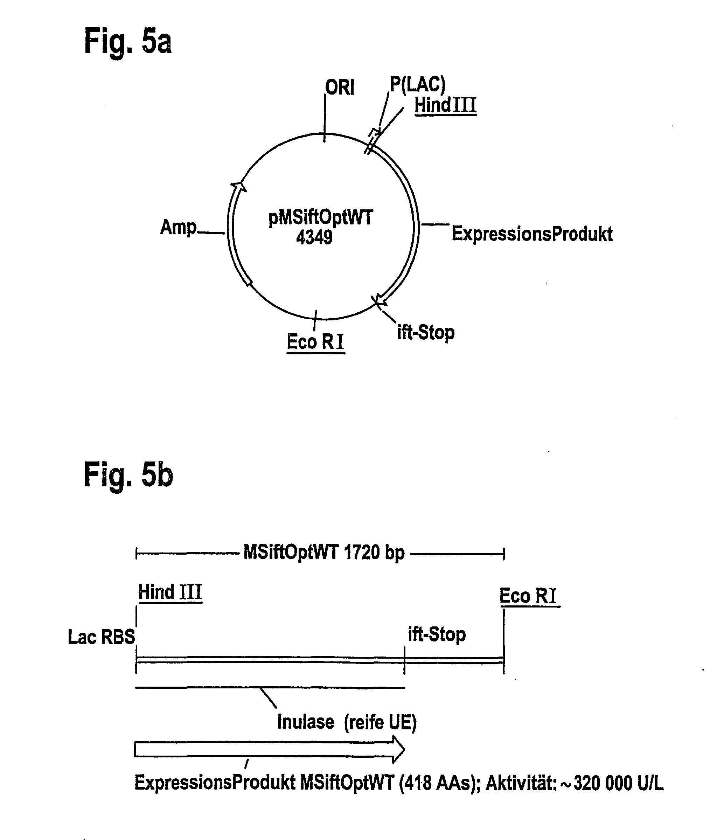

Die Genkarte des erhaltenen Expressionskonstruktes pMSiftOptWT, der Kombination aus Expressionsmatrize MSiftOptWT und dem Plasmid pUC 19, ist in Figur 5 gezeigt. Die Inulaseaktivität eines mit dem Vektor pMSiftOptWT transformierten E.coli lag bei über 320.000 U/L.

Als Expressionsprodukt wurde ein Fusionsprotein mit der in Figur 6 gezeigten Aminosäuresequenz, die 418 Aminosäuren umfasst, erhalten, wobei das Enzym mit Inulaseaktivität an der Aminosäureposition 9 ADGQQ ... beginnt.

In Figur 7 ist die DNA-Sequenz Nr. 3 eines Plasmids pMSiftOptR dargestellt, das sich von der DNA-Sequenz Nr. 2 des Plasmids pMSiftOptWT in einem Nucleotid an der Position 661 unterscheidet, indem das Nucleotid G der Sequenz Nr. 2 durch das Nucleotid A in Sequenz Nr. 3 ausgetauscht worden ist, wodurch in den entsprechenden Aminosäuresequenzen an Position 221 anstelle von G (Gly) R (Arg) eingebaut ist. Diese geringfügige Abwandlung bewirkt eine Aktivitätserhöhung auf 435.000 U/L, das heißt, eine Erhöhung um den Faktor 1,35.

Nachstehend werden die Durchführung der Experimente und die Ergebnisse eines mit den erhaltenen Expressionsprodukten (Enzymen) vorgenommenen Inulaseaktivitätstests beschrieben.

### Durchführung des Inulaseaktivitätstests

Der nachstehend beschriebene Test diente lediglich zu einer schnellen komparativen Analyse und es ist zu erwarten, dass im präparativen Maßstab unter optimierten Bedingungen wie höherer Zellzahl, effektiverer Zellaufschluss etc. die Werte für die Enzymaktivität um ein mehrfaches höher ausfallen.

Die Kultivierung der Stämme erfolgte, indem 5 ml Luria-Bertani-Medium, dem 60 µg/ml Ampicillin zugesetzt worden ist, mit einer Einzelkolonie E.coli, der mit dem jeweiligen Expressionskonstrukt (Plasmid) transformiert worden war, angeimpft wurde und 16 Stunden bei 37 °C und 170 Upm geschüttelt wurde.

Als Wirtsorganismus diente ein E.coli Stamm von Stratagene® {E.coli XL1-blue MRF' Kan: Δ(mcrA)183 Δ(mcrCB-hsdSMR-mrr)173 endA1 supE44 thi-1 recA1 gyrA96 relA1 lac [F' proAB lacl^{q}ZΔM15 Tn5 (Kan^{r})]^{c}}.

Die Inulase-II-Expression erfolgte intrazellular; die Enzymreaktion und DFA-III-Bildung erfolgte nach dem Zellaufschluss im zellfreien Extrakt.

Für den Aktivitätstest wurden jeweils 0,5 ml der vorstehend beschriebenen frischen Expressionsstamm-Kultur verwendet, wobei 0,5 ml der Kultur pelletiert, der Überstand verworfen und die Zellen in 5 ml gekühlter 0,9 %-iger NaCl-Lösung resuspendiert wurden.

Der Zellaufschluss erfolgte mittels Ultraschall (KE 76, cont. 50 %, 60 sec; Bandelin, Sonopulus HD 200).

Es wurden 1 ml der aufgeschlossenen Zellen abgenommen und in einer Tischzentrifuge 10 min pelletiert (20.000 x g). 100 µl des enzymhaltigen Überstandes wurden in 1000 µl einer 10 %-igen Inulinlösung (pH 5,5) überführt und 30 min bei 50 °C inkubiert.
Die Enzymreaktion wurde durch Erhitzen auf 100 °C für 10 min gestoppt und die Lösung in einer Tischzentrifuge zentrifugiert (10 min, 20.000 x g).
100 µl des Überstandes, der das Produkt DFA-III enthielt, wurden in 1000 µl HPLC-Laufmittel transferiert und das Produkt DFA-III mittels HPLC gemessen.

Hierbei ergaben sich für den Klon des Expressionskonstruktes pMSiftOptWT ein Messwert der Produktbildung von ungefähr 2,6 g/l, was einer Enzymaktivität von ca. 323.000 U/L entsprach (eine Unit = 1 µmol/min).

Für den Klon des Expressionskonstruktes pMSiftOptR, das sich in einem einzigen Nucleotid an der Position 661 von dem Expressionskonstrukt pMSiftOptWT unterscheidet durch Austausch von G (Sequenz Nr. 2) durch A (Sequenz Nr. 3), wurde für die Produktbildung ein Wert von 3,5 g/l DFA-III gefunden, was einer Enzymaktivität von ca. 435.000 U/L entsprach. Gegenüber dem Expressionskonstrukt pMSiftOptWT wird damit eine Steigerung um das 1,35-fache beobachtet.

Die entsprechenden Enzymaktivitäten für Klone des Expressionskonstruktes mit dem Plasmid pUC 18 sowie der Expressionsmatrize MSiftPH mit vollständiger Signalsequenz entsprechend einem Expressionsprodukt mit 477 Aminosäuren, mit verkürzter Signalsequenz entsprechend einem Expressionsprodukt mit 456 Aminosäuren beziehungsweise entsprechend einem Expressionsprodukt mit 431 Aminosäuren sowie mit der Expressionsmatrize MSiftOptWT ohne Signalsequenz lagen bei ca. 3.500, ca. 14.000, ca. 70.000 sowie ca. 120.000 U/L.

Die aus dem Arthrobacter sp. Bu0141 sowie den daraus isolierten beziehungsweise davon abgeleiteten DNA-Sequenzen, die für ein Enzym mit Inulase-II-Aktivität kodieren, erhältlichen Enzyme mit Inulase-II-Aktivität sind aufgrund ihrer hoher Thermostabilität ausgezeichnet geeignet für ein Verfahren zum enzymatischen Abbau von Inulin zur Herstellung von Difructoseanhydrid-III. Durch die aufgezeigte Möglichkeit, diese DNA-Sequenzen in allgemein verfügbare Wirtsorganismen zu transformieren und zu exprimieren, können maßgeschneidert rekombinante Mikroorganismen mit hoher Enzymaktivität erhalten werden, wobei die exprimierten Enzyme hohe Thermostabilität aufweisen, und somit effizient Inulin zu Difructoseanhydrid-III abbauen können.

## Patentansprüche

1. DNA-Sequenz kodierend für ein Enzym mit Inulase-II-Aktivität ausgewählt unter einer DNA-Sequenz mit einer Nucleotidabfolge gemäß einer der Sequenzen Nr. 1, Nr. 2 oder Nr. 3,
einer DNA-Sequenz, die den für ein Enzym mit Inulase-II-Aktivität kodierenden Bereich der Sequenzen Nr. 1, Nr. 2 oder Nr. 3 umfasst;
einer DNA-Sequenz, die ein Enzym mit Inulase-II-Aktivität kodiert, das die für die Sequenzen Nr. 1, Nr. 2 oder Nr. 3 angegebene Aminosäuresequenz umfasst; sowie
zu den DNA-Sequenzen Nrn. 1, 2 oder 3 homologen Sequenzen mit einer Identität von mehr als 90 % einschließlich des für die Signalsequenz kodierenden Bereichs und/oder für den Bereich, der für die reife Untereinheit kodiert, wobei besagte Sequenzen für ein Enzym mit Inulase-II-Aktivität kodieren, wobei das Enzym bei 60°C über 140 Stunden bei 100% Restaktivität stabil ist.

2. Vektor enthaltend eine DNA-Sequenz nach Anspruch 1.

3. Vektor nach Anspruch 2,
**dadurch gekennzeichnet,**
**dass** der Vektor ein Plasmid pUC 18 oder pUC 19 ist.

4. Plasmid ausgewählt unter Plasmiden mit der Hinterlegungsnummer DSM 13460, DSM 13461 und DSM 13462.

5. Mikroorganismus,
**dadurch gekennzeichnet,**
**dass** der Mikroorganismus eine DNA-Sequenz nach Anspruch 1, einen Vektor nach einem der Ansprüche 2 oder 3 oder ein Plasmid nach Anspruch 4 enthält.

6. Mikroorganismus nach Anspruch 5,
**dadurch gekennzeichnet,**
**dass** der Mikroorganismus ein E.coli mit der Hinterlegungsnummer DSM 13463 oder DSM 13465 ist.

7. Mikroorganismus der Spezies Arthrobacter sp. mit der Hinterlegungsnummer DSM 13464.

8. Enzym mit Inulase-II-Aktivität erhältlich durch Expression einer der DNA-Sequenzen nach Anspruch 1.

9. Verfahren zum enzymatischen Abbau von Inulin zu Difructoseanhydrid-III,
**dadurch gekennzeichnet,**
**dass** ein Enzym mit Inulase-II-Aktivität verwendet wird, das über eine der DNA-Sequenzen gemäß Anspruch 1 erhältlich ist.

10. Verfahren nach Anspruch 9,
**dadurch gekennzeichnet,**
**dass** die DNA-Sequenz in einen Mikroorganismus eingeführt und dort exprimiert wird.

11. Verfahren nach einem der Ansprüche 9 oder 10,
**dadurch gekennzeichnet,**
**dass** für das Verfahren ein Plasmid ausgewählt unter Plasmiden der Hinterlegungsnummer DSM 13460, DSM 13461 und DSM 13462 oder ein Mikroorganismus ausgewählt unter Mikroorganismen mit der Hinterlegungsnummer DSM 13463, DSM 13464 und DSM 13465 verwendet wird.

## Claims

1. DNA sequence which codes for an enzyme with inulase II activity chosen from a DNA sequence with a nucleotide sequence according to one of sequences no. 1, no. 2 or no. 3, a DNA sequence which comprises the region of sequences no. 1, no. 2 or no. 3 which codes for an enzyme with inulase II activity;
a DNA sequence which codes an enzyme with inulase II activity which comprises the amino acid sequence given for sequences no. 1, no. 2 or no. 3; and
sequences homologous to DNA sequences no. 1, 2 or 3 which have an identity of more than 72.3%, including the region which codes for the signal sequence, and/or more than 74.3% for the region which codes for the mature sub-unit, wherein said sequences code for an enzyme with inulase II activity, whereby the enzyme is stable at 60°C for 140 hours with 100% residual activity.

2. Vector comprising a DNA sequence according to claim 1.

3. Vector according to claim 2,
**characterized in that**
the vector is a plasmid pUC 18 or pUC 19.

4. Plasmid chosen from plasmids with deposit number DSM 13460, DSM 13461 and DSM 13462.

5. Microorganism
**characterized in that**
the microorganism contains a DNA sequence according to claim 1, a vector according to one of claims 2 or 3 or a plasmid according to claim 4.

6. Microorganism according to claim 5,
**characterized in that**
the microorganism is an E. coli with deposit number DSM 13463 or DSM 13465.

7. Microorganism of the species Arthrobacter sp. with deposit number DSM 13464.

8. Enzyme with inulase II activity, obtainable by expression of one of the DNA sequences according to claim 1.

9. Process for the enzymatic decomposition of inulin to difructose anhydride III,
**characterized in that**
there is used an enzyme with inulase II activity which is obtainable via one of the DNA sequences according to claim 1.

10. Process according to claim 9,
**characterized in that**
the DNA sequence is introduced into a microorganism and expressed there.

11. Process according to one of claims 9 or 10,
**characterized in that**
a plasmid chosen from plasmids with deposit number DSM 13460, DSM 13461 and DSM 13462 or a microorganism chosen from microorganisms with deposit number DSM 13463, DSM 13464 and DSM 13465 is used for the process.

## Revendications

1. Séquence d'ADN codant pour une enzyme avec activité d'inulase II, choisie parmi une séquence d'ADN avec une succession de nucléotides selon l'une des séquences N°1, N°2 ou N°3,
une séquence d'ADN, qui comprend le domaine codant pour une enzyme avec activité d'inulase II des séquences N°1, N°2 ou N°3 ;
une séquence d'ADN, qui code pour une enzyme avec activité d'inulase II, qui comprend la séquence des acides aminés données pour les séquences N°1, N°2 ou N°3 ; ainsi que
des séquences homologues aux séquences N°1, N°2 ou N°3, avec une identité de plus de 90%, y compris le domaine codant pour la séquence signal et/ou le domaine, qui code pour la sous-unité mature, où les séquences considérées codent pour une enzyme avec activité d'inulase II, où l'enzyme est stable à 60°C pendant 140 secondes avec une activité résiduelle de 100%.

2. Vecteur contenant une séquence d'ADN selon la revendication 1.

3. Vecteur selon la revendication 2, **caractérisé en ce que** le vecteur est un plasmide pUC 18 ou pUC 19.

4. Plasmide choisi parmi les plasmides avec le numéro de dépôt DSM 13460, DSM 13461 et DSM 13462.

5. Microorganisme, **caractérisé en ce que** le microorganisme contient une séquence d'ADN selon la revendication 1, un vecteur selon l'une quelconque des revendications 2 ou 3 ou un plasmide selon la revendication 4.

6. Microorganisme selon la revendication 5, **caractérisé en ce que** le microorganisme est un E. *coli* avec le numéro de dépôt DSM 13463 ou DSM 13465.

7. Microorganisme de l'espèce *Arthrobacter* sp. avec le numéro de dépôt DSM 13464.

8. Enzyme avec activité d'inulase II, disponible par expression d'une des séquences d'ADN selon la revendication 1.

9. Procédé de dégradation enzymatique d'inuline en anhydride de difructose-III, **caractérisé en ce que** l'on utilise une enzyme avec activité d'inulase II, qui est obtenue par une des séquences d'ADN selon la revendication 1.

10. Procédé selon la revendication 9, **caractérisé en ce que** la séquence d'ADN est introduite dans un microorganisme et y est exprimée.

11. Procédé selon l'une quelconque des revendications 9 ou 10, **caractérisé en ce que** l'on utilise pour le procédé, un plasmide choisi parmi les plasmides de numéro de dépôt DSM 13460, DSM 13461 et DSM 13462 ou un microorganisme choisi parmi les microorganismes avec le numéro de dépôt DSM 13463, DSM 13464 et DSM 13465.


### SEQUENZPROTOKOLL-Teil

<110> Nordzucker AG
<120> Verfahren zur mikrobiellen Herstellung von Difructoseanhydrid-III, dafür einsetzbarer Mikroorganismus sowie Enzym mit Inulase-II-Aktivität und dafür kodierende DNA-Sequenzen
<130> 100 24 569.2
<140> 9904638
   <141> 2000-05-19
<160> 3
<170> PatentIn Ver. 2.1
<210> 1
   <211> 1884
   <212> DNA
   <213> Arthrobacter sp.
<220>
   <221> CDS
   <222> (35)..(1384)
<220>
   <221> mat_peptide
   <222> (155)..(1384)
<400> 1
   ctgcagcagt cttatcccat acaaaggaga cccc gtg gta act ggc aag aat cta 55
                                         Val Val Thr Gly Lys Asn Leu
                                         -40                 -35
   gac aaa gcg aat cca agc cgc cgt cgg ctg atc ggc gcc gga gcc gcc   103
   Asp Lys Ala Asn Pro Ser Arg Arg Arg Leu Ile Gly Ala Gly Ala Ala
               -30                 -25                 -20
   gga acc ctg gcg gct gcc ttg acc ctc ggg acg atg cag aac gcc aat   151
   Gly Thr Leu Ala Ala Ala Leu Thr Leu Gly Thr Met Gln Asn Ala Asn
           -15                 -10                 -5
   gcg gcc gac ggc cag caa ggt acc ccc ctc aat tcg ccc aac acg tac   199
   Ala Ala Asp Gly Gln Gln Gly Thr Pro Leu Asn Ser Pro Asn Thr Tyr
    -1   1               5                  10                  15
   gac gta acc aca tgg agg atc aag gca cac ccg gac gtc acc gcg cag   247
   Asp Val Thr Thr Trp Arg Ile Lys Ala His Pro Asp Val Thr Ala Gln
                    20                  25                  30
   tcc gac att ggg gcg gtc atc aac gac atc atc gcc gac atc aag caa   295
   Ser Asp Ile Gly Ala Val Ile Asn Asp Ile Ile Ala Asp Ile Lys Gln
                35                  40                  45
   cgg cag acg tca ccg gac gcg cgt ccc gga gcc gcg atc att atc cca    343
   Arg Gln Thr Ser Pro Asp Ala Arg Pro Gly Ala Ala Ile Ile Ile Pro
            50                  55                  60
   ccg ggc gac tac gac ctg cac acc cag gtc gtc gtc gac ata agt tac    391
   Pro Gly Asp Tyr Asp Leu His Thr Gln Val Val Val Asp Ile Ser Tyr
        65                  70                  75
   ctg aca atc gcg ggc ttc ggg cat ggc ttc ttc tcc cga agc atc ctc    439
   Leu Thr Ile Ala Gly Phe Gly His Gly Phe Phe Ser Arg Ser Ile Leu
    80                  85                  90                  95
   gac aac tcg aac ccg acc gga tgg cag aac ctc caa ccc gga gca agc    487
   Asp Asn Ser Asn Pro Thr Gly Trp Gln Asn Leu Gln Pro Gly Ala Ser
                   100                 105                 110
   cac atc cgc gtc ctg acc tct ccg agc gcg ccc cag gca ttc ctc gtc    535
   His Ile Arg Val Leu Thr Ser Pro Ser Ala Pro Gln Ala Phe Leu Val
               115                 120                 125
   cgc cgg aca ggg gat ccc cgt ctt tca gga atc gtg ttc cgg gac ttc    583
   Arg Arg Thr Gly Asp Pro Arg Leu Ser Gly Ile Val Phe Arg Asp Phe
           130                 135                 140
   tgc ctc gac gga gtc ggc ttc acc ccc gac aag aac agc tac cac aac    631
   Cys Leu Asp Gly Val Gly Phe Thr Pro Asp Lys Asn Ser Tyr His Asn
       145                 150                 155
   ggc aag acc gga atc gaa gtc gcc tcc gac aac gac tcc ttc cac atc    679
   Gly Lys Thr Gly Ile Glu Val Ala Ser Asp Asn Asp Ser Phe His Ile
   160                 165                 170                 175
   acc ggc atg gga ttc gtc tac ctc gaa cat gcc ctg atc gtg cgc ggc    727
   Thr Gly Met Gly Phe Val Tyr Leu Glu His Ala Leu Ile Val Arg Gly
                   180                 185                 190
   gcc gac gcg ctc cgc gtc aac gac aac atg atc gcc gaa tgc ggc aac    775
   Ala Asp Ala Leu Arg Val Asn Asp Asn Met Ile Ala Glu Cys Gly Asn
               195                 200                 205
   tgc gtc gag ctc acc ggg gcc ggg cag gcc aca att gtc agc ggc aat    823
   Cys Val Glu Leu Thr Gly Ala Gly Gln Ala Thr Ile Val Ser Gly Asn
           210                 215                 220
   cac atg ggc gcc ggc cct gac ggg gta acc ctc ctg gcc gag aac cac    871
   His Met Gly Ala Gly Pro Asp Gly Val Thr Leu Leu Ala Glu Asn His
       225                 230                 235
   gag ggc ctc ctc gtc acc ggc aac aac ctc ttc cca cgc ggc cgc agc    919
   Glu Gly Leu Leu Val Thr Gly Asn Asn Leu Phe Pro Arg Gly Arg Ser
   240                 245                 250                 255
   ctc atc gaa ctc acc ggc tgc aac cgg tcc tca gtc tcc tcg aac agg    967
   Leu Ile Glu Leu Thr Gly Cys Asn Arg Ser Ser Val Ser Ser Asn Arg
                   260                 265                 270
   ctc cag ggc ttt tac ccg ggc atg ctc cgc ctg ctg aac ggc tgc aag    1015
   Leu Gln Gly Phe Tyr Pro Gly Met Leu Arg Leu Leu Asn Gly Cys Lys
               275                 280                 285
   gag aac ctc atc acg gcc aac cac atc cgc cgg acc aac gag ggg tac    1063
   Glu Asn Leu Ile Thr Ala Asn His Ile Arg Arg Thr Asn Glu Gly Tyr
           290                 295                 300
   ccg ccg ttc atc ggc cgc ggc aac ggc ctc gac gac ctc tac ggc gtc    1111
   Pro Pro Phe Ile Gly Arg Gly Asn Gly Leu Asp Asp Leu Tyr Gly Val
       305                 310                 315
   gtc cac atc gcg gga gac aac aac ctc atc tcg gac aac ctc ttc gcc    1159
   Val His Ile Ala Gly Asp Asn Asn Leu Ile Ser Asp Asn Leu Phe Ala
   320                 325                 330                 335
   tac aac gtc ccg ccc ggc aac atc gcc ccc gcc ggc gcc cag ccg acc    1207
   Tyr Asn Val Pro Pro Gly Asn Ile Ala Pro Ala Gly Ala Gln Pro Thr
                   340                 345                 350
   cag atc ctg atc gcc ggc gga gac gcc aac gtg gtg gcg ctc aac cac    1255
   Gln Ile Leu Ile Ala Gly Gly Asp Ala Asn Val Val Ala Leu Asn His
               355                 360                 365
   gtg gtc agc gac gtc gct tcc cag cac gtc gtt ctg gac gca tcc acc    1303
   Val Val Ser Asp Val Ala Ser Gln His Val Val Leu Asp Ala Ser Thr
           370                 375                 380
   act cac tcg aaa gtg ctc gac agc ggt acc gcc tcc cag atc acc tcg    1351
   Thr His Ser Lys Val Leu Asp Ser Gly Thr Ala Ser Gln Ile Thr Ser
       385                 390                 395
   tac agc acg gac acc gct atc cgg ccg acc ccc tgacaggcgg agagcagctt 1404
   Tyr Ser Thr Asp Thr Ala Ile Arg Pro Thr Pro
   400                 405                 410
   ctcggaaacc accggacgcg ccaagggcat ttcttatgtt ggggcccgga ccaatcggtg 1464
   atatcgcggg gagcctcagc ggtccttgag aggctccccg atcaattcgg gctgccggtt 1524
   gctccagtcg tggaagtagg gagcggcgcc gtggtggtgc ttgttgttgt actcctgggc 1584
   aagacccagt gcaccttcga gcccggggaa gacccggtct ttggtgtgat cagcgcatct 1644
   gacgaggaaa ccgagccccc taaagccgta gcactgggtt acataagcgg gtcgagtcga 1704
   aatgtccccc ttggtgtcgt tccgccctcc gacggggccc gcttagatgg ttctatctcc 1764
   ggaatcctga tctacctcag tcactggtga tttgatccat gtgacgacca cactcacccc 1824
   gccgtcctcg tcccgttcgg tctcgatttc aatctcggaa gccgacgccc caataagctt 1884
<210> 2
<211> 1737
   <212> DNA
   <213> Arthrobacter sp.
<220>
   <221> CDS
   <222> (1)..(255)
<220>
   <221> mat_peptide
   <222> (25)..(255)
<400> 2
   atg acc atg att acg cca agc ttg gcc gac ggc cag caa ggt acc ccc    48
   Met Thr Met Ile Thr Pro Ser Leu Ala Asp Gly Gln Gln Gly Thr Pro
               -5              -1   1               5
   ctc aat tcg ccc aac acg tac gac gta acc aca tgg agg atc aag gca    96
   Leu Asn Ser Pro Asn Thr Tyr Asp Val Thr Thr Trp Arg Ile Lys Ala
        10                  15                  20
   cac ccg gac gtc acc gcg cag tcc gac att ggg gcg gtc atc aac gac   144
   His Pro Asp Val Thr Ala Gln Ser Asp Ile Gly Ala Val Ile Asn Asp
    25                  30                  35                  40
   atc atc gcc gac atc aag caa cgg cag acg tca ccg gac gcg cgt ccc   192
   Ile Ile Ala Asp Ile Lys Gln Arg Gln Thr Ser Pro Asp Ala Arg Pro
                    45                  50                  55
   gga gcc gcg atc att atc cca ccg ggc gac tac gac ctg cac acc cag   240
   Gly Ala Ala Ile Ile Ile Pro Pro Gly Asp Tyr Asp Leu His Thr Gln
               60                  65                  70
   gtc gtc gtc gac ata agttacctga caatcgcggg cttcgggcat ggcttcttct   295
   Val Val Val Asp Ile
           75
   cccgaagcat cctcgacaac tcgaacccga ccggatggca gaacctccaa cccggagcaa 355
   gccacatccg cgtcctgacc tctccgagcg cgccccaggc attcctcgtc cgccggacag 415
   gggatccccg tctttcagga atcgtgttcc gggacttctg cctcgacgga gtcggcttca 475
   cccccgacaa gaacagctac cacaacggca agaccggaat cgaagtcgcc tccgacaacg 535
   actccttcca catcaccggc atgggattcg tctacctcga acatgccctg atcgtgcgcg 595
   gcgccgacgc gctccgcgtc aacgacaaca tgatcgccga atgcggcaac tgcgtcgagc 655
   tcaccggggc cgggcaggcc acaattgtca gcggcaatca catgggcgcc ggccctgacg 715
   gggtaaccct cctggccgag aaccacgagg gcctcctcgt caccggcaac aacctcttcc 775
   cacgcggccg cagcctcatc gaactcaccg gctgcaaccg gtcctcagtc tcctcgaaca 835
   ggctccaggg cttttacccg ggcatgctcc gcctgctgaa cggctgcaag gagaacctca 895
   tcacggccaa ccacatccgc cggaccaacg aggggtaccc gccgttcatc ggccgcggca 955
   acggcctcga cgacctctac ggcgtcgtcc acatcgcggg agacaacaac ctcatctcgg 1015
   acaacctctt cgcctacaac gtcccgcccg gcaacatcgc ccccgccggc gcccagccga 1075
   cccagatcct gatcgccggc ggagacgcca acgtggtggc gctcaaccac gtggtcagcg 1135
   acgtcgcttc ccagcacgtc gttctggacg catccaccac tcactcgaaa gtgctcgaca 1195
   gcggtaccgc ctcccagatc acctcgtaca gcacggacac cgctatccgg ccgaccccct 1255
   gacaggcgga gagcagcttc tcggaaacca ccggacgcgc caagggcatt tcttatgttg 1315
   gggcccggac caatcggtga tatcgcgggg agcctcagcg gtccttgaga ggctccccga 1375
   tcaattcggg ctgccggttg ctccagtcgt ggaagtaggg agcggcgccg tggtggtgct 1435
   tgttgttgta ctcctgggca agacccagtg caccttcgag cccggggaag acccggtctt 1495
   tggtgtgatc agcgcatctg acgaggaaac cgagccccct aaagccgtag cactgggtta 1555
   cataagcggg tcgagtcgaa atgtccccct tggtgtcgtt ccgccctccg acggggcccg 1615
   cttagatggt tctatctccg gaatcctgat ctacctcagt cactggtgat ttgatccatg 1675
   tgacgaccac actcaccccg ccgtcctcgt cccgttcggt ctcgatttca atctcggaat 1735
   tc 1737
<210> 3
   <211> 1737
   <212> DNA
   <213> Arthrobacter sp.
<400> 3
   atgaccatga ttacgccaag cttggccgac ggccagcaag gtacccccct caattcgccc 60
   aacacgtacg acgtaaccac atggaggatc aaggcacacc cggacgtcac cgcgcagtcc 120
   gacattgggg cggtcatcaa cgacatcatc gccgacatca agcaacggca gacgtcaccg 180
   gacgcgcgtc ccggagccgc gatcattatc ccaccgggcg actacgacct gcacacccag 240
   gtcgtcgtcg acataagtta cctgacaatc gcgggcttcg ggcatggctt cttctcccga 300
   agcatcctcg acaactcgaa cccgaccgga tggcagaacc tccaacccgg agcaagccac 360
   atccgcgtcc tgacctctcc gagcgcgccc caggcattcc tcgtccgccg gacaggggat 420
   ccccgtcttt caggaatcgt gttccgggac ttctgcctcg acggagtcgg cttcaccccc 480
   gacaagaaca gctaccacaa cggcaagacc ggaatcgaag tcgcctccga caacgactcc 540
   ttccacatca ccggcatggg attcgtctac ctcgaacatg ccctgatcgt gcgcggcgcc 600
   gacgcgctcc gcgtcaacga caacatgatc gccgaatgcg gcaactgcgt cgagctcacc 660
   agggccgggc aggccacaat tgtcagcggc aatcacatgg gcgccggccc tgacggggta 720
   accctcctgg ccgagaacca cgagggcctc ctcgtcaccg gcaacaacct cttcccacgc 780
   ggccgcagcc tcatcgaact caccggctgc aaccggtcct cagtctcctc gaacaggctc 840
   cagggctttt acccgggcat gctccgcctg ctgaacggct gcaaggagaa cctcatcacg 900
   gccaaccaca tccgccggac caacgagggg tacccgccgt tcatcggccg cggcaacggc 960
   ctcgacgacc tctacggcgt cgtccacatc gcgggagaca acaacctcat ctcggacaac 1020
   ctcttcgcct acaacgtccc gcccggcaac atcgcccccg ccggcgccca gccgacccag 1080
   atcctgatcg ccggcggaga cgccaacgtg gtggcgctca accacgtggt cagcgacgtc 1140
   gcttcccagc acgtcgttct ggacgcatcc accactcact cgaaagtgct cgacagcggt 1200
   accgcctccc agatcacctc gtacagcacg gacaccgcta tccggccgac cccctgacag 1260
   gcggagagca gcttctcgga aaccaccgga cgcgccaagg gcatttctta tgttggggcc 1320
   cggaccaatc ggtgatatcg cggggagcct cagcggtcct tgagaggctc cccgatcaat 1380
   tcgggctgcc ggttgctcca gtcgtggaag tagggagcgg cgccgtggtg gtgcttgttg 1440
   ttgtactcct gggcaagacc cagtgcacct tcgagcccgg ggaagacccg gtctttggtg 1500
   tgatcagcgc atctgacgag gaaaccgagc cccctaaagc cgtagcactg ggttacataa 1560
   gcgggtcgag tcgaaatgtc ccccttggtg tcgttccgcc ctccgacggg gcccgcttag 1620
   atggttctat ctccggaatc ctgatctacc tcagtcactg gtgatttgat ccatgtgacg 1680
   accacactca ccccgccgtc ctcgtcccgt tcggtctcga tttcaatctc ggaattc    1737